# EUROPEAN PATENT SPECIFICATION

(11) **EP 0 420 532 B1**
(45) Date of publication and mention of the grant of the patent: **08.06.1994**
(21) Application number: 90310389.3
(22) Date of filing: 21.09.1990
(51) Int. Cl.: C07D 311/08

(54) **Process for producing 3,4-dihydrocoumarin derivatives**
Verfahren zur Herstellung von 3,4-Dihydrocumarinderivaten
Procédé de préparation de dérivés de 3,4-dihydrocoumarines

(30) Priority: 25.09.1989 JP 249748/89; 25.09.1989 JP 249749/89
(43) Date of publication of application: 03.04.1991
(73) Proprietor: SUMITOMO CHEMICAL COMPANY LIMITED, Osaka-shi, Osaka 541 (JP)
(72) Inventor: Shirafuji, Tamio, Niihama-shi, Ehime (JP); SAKAI, Kiyomi, Niihama-shi, Ehime (JP); Okusako, Kensen, Niihama-shi, Ehime (JP); Kawata, Itaru, Niihama-shi, Ehime (JP); Simazu, Yasumoto, Ibaraki-shi, Osaka (JP); Suzuta, Tetuya, Kobe-shi, Hyogo (JP)
(74) Representative: Diamond, Bryan Clive

(56) References cited:
- US-A- 3 442 910
- CHEMICAL ABSTRACTS, vol. 104, no. 11, 17th March 1986, page 647, abstract no.88436w, Columbus, Ohio, US; & JP-A-60 181 082 (SUMITOMO) 14-09-1985
- JOURNAL OF THE AMERICAN CHEMICAL SOCIETY, vol. 62, no. 2, 7th February 1940,pages 283-287, Washington, DC, US; P.L. DE BENNEVILLE et al.: "Thehydrogenation of coumarin and related compounds"

## Description

The present invention relates to an improvement in a process for the preparation of a 3,4-dihydrocoumarin derivative from a 3-(2-cyclohexanoyl)propionic acid ester derivative.

3,4-Dihydrocoumarin derivatives are important compounds, e.g., in the perfume industry.

In a conventionally employed method for producing a 3,4-dihydrocoumarin derivative, a 3-(2-cyclohexanoyl)propionic acid ester derivative is heated in the presence of a hydrogenation-dehydrogenation catalyst such as palladium to perform ring formation and dehydrogenation. In the above reaction, a coumarin derivative is formed as a by- product. This by-product coumarin derivative is separated from the reaction mixture and then partially hydrogenated with hydrogen in the presence of a fresh hydrogenation-dehydrogenation catalyst so as to convert the coumarin derivative into a dihydrocoumarin derivative, and the resulting dihydrocoumarin derivative is recovered (as described, e.g., in U.S. Patent 3,442,910 and J. Am. Chem. Soc., vol. 62, pp. 283-287 (1940)).

The catalyst for use in the partial hydrogenation reaction may be one obtained by regenerating the catalyst that has been used in the ring formation and dehydrogenation reaction. The regeneration of the catalyst is accomplished by (1) subjecting the catalyst to hydrogen reduction at a high temperature (Shokubai Sekkei (Catalyst Design), edited by Shokubai Gakkai, published by Kodansha, Japan) or (2) subjecting the catalyst to cracking at a high temperature (as described, e.g., in U.S. Patents 2,506,307 and 2,532,615).

The above-described conventional methods are defective in that both the by-product coumarin derivative and the catalyst should be separated from the reaction mixture, and that it is necessary to conduct the regeneration of the catalyst at a high temperature (about 480 to 600°C) in either of the above methods (1) and (2). Thus, the separation of the reaction product and the regeneration of the catalyst necessitates difficult procedures and are uneconomical. In addition, the regeneration method (1) above is dangerous because hydrogen is used at a high temperature.

The present inventors have now devised a highly economical process for producing a 3,4-dihydrocoumarin derivative. They have found that if, after a 3-(2-cyclohexanoyl)propionic acid ester derivative is heated in the presence of a hydrogenation-dehydrogenation catalyst to perform ring formation and dehydrogenation with formation of a coumarin derivative as a by-product, the partial hydrogenation of the by-product coumarin derivative is conducted using hydrogen with the coumarin derivative being kept unseparated from the reaction mixture, the partial hydrogenation reaction proceeds at a higher rate than partial hydrogenation conducted after the coumarin derivative is separated from the reaction mixture.

The present inventors have also found that by bringing the reaction mixture, as it is, that has undergone ring formation and dehydrogenation into contact with oxygen, the catalyst in the reaction mixture is regenerated, and that even if the resulting reaction mixture is then subjected, as it is, to partial hydrogenation using hydrogen, only the by-product coumarin derivative is partially hydrogenated. The present invention has been completed based on these findings.

It is, therefore, an object of the present invention to provide an efficient and economical process for producing a 3,4-dihydrocoumarin derivative.

The present invention provides a process for producing a 3,4-dihydrocoumarin derivative represented by formula (II): 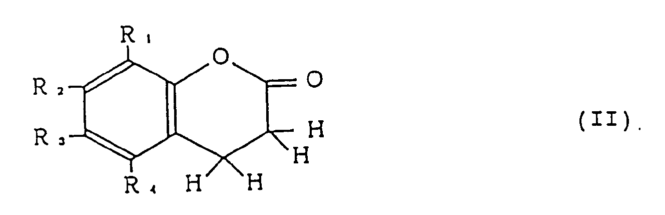
wherein R₁ to R₄ each represents a hydrogen atom, a methyl group or an ethyl group, provided that at least two of R₁ to R₄ each represents a hydrogen atom,
which process comprises the steps of: (1) heating a 3-(2-cyclohexanoyl)propionic acid ester derivative represented by formula (I): 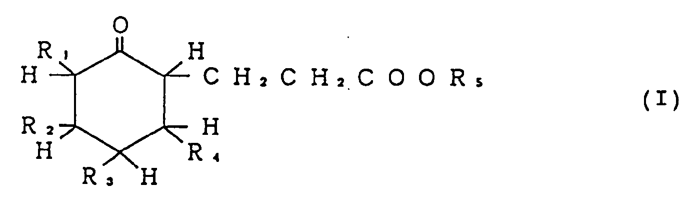
wherein R₁ to R₄ are as defined above, and R₅ represents an alkyl group having 1 to 4 carbon atoms,
in the presence of at least one solid metal catalyst selected from platinum, palladium, iridium, rhodium and nickel, thereby to allow the compound of formula (I) to undergo ring formation and dehydrogenation to yield the 3,4-dihydrocoumarin derivative of formula (II) and, as a by-product, a coumarin derivative represented by formula (III): 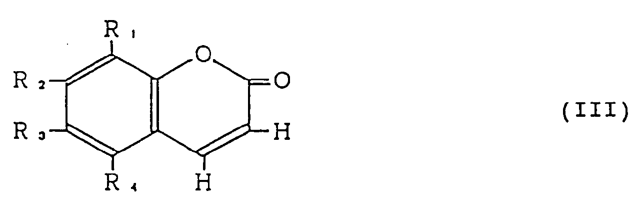
wherein R₁ to R₄ are as defined above; and
(2) partially hydrogenating the coumarin derivative of formula (III),
the partial hydrogenation of the byproduct coumarin derivative of formula (III) being conducted, while the coumarin derivative is kept unseparated from the reaction mixture that has undergone the ring formation and dehydrogenation,
by adding a catalyst which is as defined above to the reaction mixture, or bringing the reaction mixture into contact with oxygen to activate the catalyst used in the ring formation and dehydrogenation; and then bringing the resulting reaction mixture into contact with hydrogen thereby to partially hydrogenate the coumarin derivative of formula (III) so as to convert the coumarin derivative of formula (III) to the 3,4-dihydrocoumarin derivative of formula (II).

The 3-(2-cyclohexanoyl)propionic acid ester derivative used in the present invention is represented by formula (I) given above. Examples of this compound include: methyl 3-(2-cyclohexanoyl)propionate (which is preferred), butyl 3-(2-cyclohexanoyl)propionate, methyl 3-(3-methyl-2-cyclohexanoyl)propionate, methyl 3-(5-methyl-2-cyclohexanoyl)propionate, propyl 3-(4-ethyl-2-cyclohexanoyl)propionate, ethyl 3-(3,4-dimethyl-2-cyclohexanoyl)propionate, methyl 3-(3,5-diethyl-2-cyclohexanoyl)propionate, and propyl 3-(3-ethyl-6-methyl-2-cyclohexanoyl)propionate.

The catalyst used in the present invention is a heterogeneous catalyst which comprises at least one element selected from platinum, palladium, iridium, rhodium and nickel. The catalyst is preferably supported on at least one carrier selected from compounds of Group II, III, and IV elements of the Periodic Table, such as carbon, alumina, silica gel, zeolite, magnesium oxide, barium sulfate or calcium carbonate.

These catalysts may be prepared by known methods, for example, by the impregnation-fixation technique (Shokubai Jikken Manual (Catalyst Experiment Manual), edited by Shokubai Gakkai, published by Maki Shoten, Japan) in which a carrier is impregnated with a metal and the resulting carrier is subjected to hydrogen reduction at a high temperature. However, a commercially available catalyst may also be used.

The amount of the catalyst used for the ring formation and dehydrogenation is generally from 0.1 to 5% by weight, preferably from 0.5 to 2% by weight, based on the amount of the 3-(2-cyclohexanoyl)propionic acid ester derivative, because too small an amount of the catalyst results in a low reaction rate, whereas too large an amount thereof is costly although the reaction rate is high.

The ring formation and dehydrogenation reaction of the 3-(2-cyclohexanoyl)propionic acid ester derivative may be carried out generally at from 100 to 350°C, preferably at from 230 to 260°C. Temperatures outside the above range are not preferred because too low a temperature results in a low reaction rate, while a temperature exceeding 350°C tends to cause the starting material and/or the product to decompose.

The ring formation and dehydrogenation reaction may be conducted by using a solvent. Examples of the solvent include phenyl ether, benzyl ether, methyl α-naphthyl ether, ethylnaphthalene, dimethylbiphenyl, dodecane, tetradecane, tetralin, acetophenone, phenyl propyl ketone, methyl benzoate or dimethyl glutamate. The amount of the solvent is generally from 0.5 to 10 times, preferably from 1 to 5 times, the amount of the 3-(2-cyclohexanoyl)propionic acid derivative.

The ring formation and dehydrogenation reaction can be carried out by heating the 3-(2-cyclohexanoyl)propionic acid ester derivative and the catalyst, along with a solvent if required, at a predetermined temperature generally for 5 to 45 hours, preferably for 15 to 30 hours.

As a result of the reaction, a 3,4-dihydrocoumarin compound and a by-product coumarin derivative are obtained in yields of from 70 to 80% and from 3 to 20%, respectively. Besides these compounds, o-ethylphenol, methyl dihydrocinnamate, etc. result as by-products.

After the ring formation and dehydrogenation reaction, the resulting reaction mixture per se can be brought into contact with hydrogen to conduct the partial hydrogenation reaction of the by-product coumarin derivative contained in the reaction mixture. For this reaction, a catalyst is newly added in an amount of generally from 0.05 to 5% by weight, preferably from 0.1 to 2% by weight, based on the amount of the 3-(2-cyclohexanoyl)propionic acid ester derivative used in the ring formation and dehydrogenation reaction.

Alternatively, in place of newly adding a catalyst, the catalyst that has been used for the ring formation and dehydrogenation reaction and is contained in the resulting reaction mixture may be activated and then used to conduct the partial hydrogenation reaction. In order to activate the catalyst, oxygen gas may be bubbled through the reaction mixture at a temperature of from 0 to 200°C, preferably from 10 to 150°C, more preferably from 25 to 85°C, for a period of from 10 minutes to 10 hours, preferably from 30 minutes to 5 hours, so that the reaction mixture comes into sufficient contact with oxygen. The oxygen gas may be pure oxygen or a mixed gas composed of oxygen and other gas(es), such as air and nitrogen gas containing 0.5% of oxygen, and the contact may be carried out either at ordinary pressure or at an elevated pressure.

After the activation of the catalyst, the atmosphere in the reactor is replaced with an inert gas such as nitrogen to prepare for partial hydrogenation reaction using hydrogen.

The partial hydrogenation reaction of the by-product coumarin derivative contained in the reaction mixture may be carried out by bringing the reaction mixture, in which a catalyst is newly added or the catalyst has been activated, into contact with hydrogen at a temperature of from 80 to 160°C, preferably from 100 to 150°C, at ordinary pressure or an elevated pressure (generally from 1 to 10 kg/cm²G, preferably from 2 to 5 kg/cm²G) until hydrogen comes to be no longer taken up by the reaction mixture. It is preferred that the reaction mixture is further made in contact with hydrogen for 10 minutes to 3 hours, preferably from 15 minutes to 2 hours, after hydrogen comes to be no longer taken up by the reaction mixture. This partial hydrogenation reaction proceeds at a high rate as compared with the case in which the byproduct coumarin derivative is subjected to partial hydrogenation after being separated from the reaction mixture.

The reaction mixture to be subjected to the partial hydrogenation reaction may contain the solvent that was used for the ring formation and dehydrogenation reaction, or a solvent may be newly added to the reaction mixture for the partial hydrogenation. The amount of the solvent newly added is generally from 0.5 to 10 times, preferably from 1 to 5 times, the amount of the 3-(2-cyclohexanoyl)propionic acid derivative.

Through this partial hydrogenation, the by-product coumarin derivative can be hydrogenated into 3,4-dihydrocoumarin derivative substantially completely, without changing the 3,4-dihydrocoumarin derivative at all that has been formed by ring formation and dehydrogenation reaction.

After completion of the above reaction, the 3,4-dihydrocoumarin derivative can be easily isolated by ordinary techniques. That is, the compound can be isolated by distillation after separation of the catalyst.

As described above, the process of the present invention needs no difficult procedures and is able to produce a 3,4-dihydrocoumarin derivative efficiently at high reaction rates.

The present invention will be explained in more detail by reference to the following examples, wherein the percentages are by weight.

### EXAMPLE 1

300 Grams of methyl 3-(2-cyclohexanoyl)propionate was mixed with 1.5 g of a catalyst consisting of active carbon and 5% by weight of palladium supported thereon. This mixture was placed in an autoclave, which was then replaced with nitrogen. The mixture in the autoclave was then heated at 250°C for 23 hours with stirring at 300 rpm.

The resulting reaction mixture was analyzed by gas chromatography, and it was found that the conversion of the methyl 3-(2-cyclohexanoyl)propionate was 99.8% and the yields of 3,4-dihydrocoumarin and coumarin were 69% and 5%, respectively.

Thereafter, 0.3 g of a catalyst consisting of active carbon and 5% by weight of palladium supported thereon was added to the reaction mixture (the amount of the catalyst being 0.1% by weight based on the amount of the starting methyl 3-(2-cyclohexanoyl)propionate used). The temperature of the resulting reaction mixture in the autoclave was adjusted to 120°C, and partial hydrogenation reaction was carried out with stirring at 800 rpm while the inside of the autoclave was maintained at 2 kg/cm² with hydrogen. The time required to complete the partial hydrogenation reaction (the time required for hydrogen to come to be no longer absorbed) was 50 minutes. This 50-minute period include a 20-minute period (hereinafter, referred to as an aging period) during which the reaction was allowed to further proceed after hydrogen absorption came to no longer take place.

As a result, the yield of 3,4-dihydrocoumarin formed by the partial hydrogenation reaction was 99% based on the coumarin, and the overall yield of the product based on the methyl 3-(2-cyclohexanoyl)propionate was 74%.

### COMPARATIVE EXAMPLE 1

In an autoclave were placed 210 g of 3,4-dihydrocoumarin and 15 g of coumarin both isolated from a reaction mixture. Thereto was added 0.3 g of a catalyst consisting of active carbon and 5% by weight of palladium supported on the active carbon. The resulting mixture in the autoclave was kept at 120°C, and partial hydrogenation reaction was carried out with stirring at 800 rpm while the inside of the autoclave was maintained at 2 kg/cm² with hydrogen. The time required to complete the partial hydrogenation reaction was 4 hours (including a 20-minute aging period).

The yield of 3,4-dihydrocoumarin formed by the partial hydrogenation reaction was 87% based on the coumarin.

### EXAMPLE 2

300 Grams of methyl 3-(2-cyclohexanoyl)propionate was mixed with 1.5 g of a catalyst consisting of active carbon and 5% by weight of palladium supported thereon. This mixture was placed in an autoclave, which was then replaced with nitrogen. The mixture in the autoclave was then heated at 250°C for 23 hours with stirring at 300 rpm.

The resulting reaction mixture was analyzed by gas chromatography, and it was found that the conversion of the methyl 3-(2-cyclohexanoyl)propionate was 99.9% and the yields of 3,4-dihydrocoumarin and coumarin were 70% and 5%, respectively. Besides these compounds, methyl dihydrocinnamate and other compounds were found to have been formed as by-products.

The temperature of the reaction mixture was then adjusted to 80°C, and nitrogen gas containing 1% of oxygen was bubbled through the reaction mixture for 30 minutes with stirring at 800 rpm. Thereafter, the inside of the autoclave was replaced with nitrogen gas.

The resulting reaction mixture in the autoclave was heated at 120°C, and partial hydrogenation reaction was carried out with stirring at 800 rpm while the inside of the autoclave was maintained at 2 kg/cm² with hydrogen. The time required to complete the partial hydrogenation reaction was 2 hours (including a 20-minute aging period).

As a result, the yield of 3,4-dihydrocoumarin formed by the partial hydrogenation reaction was 90% based on the coumarin and the overall yield of the product based on the methyl 3-(2-cyclohexanoyl)propionate was 75%.

### EXAMPLE 3

The ring formation and dehydrogenation reaction of methyl 3-(2-cyclohexanoyl)propionate was carried out in the same manner as in Example 2. The temperature of the reaction mixture was then adjusted to 80°C, and air was bubbled through the reaction mixture for 30 minutes with stirring at 800 rpm. Partial hydrogenation reaction was then effected in the same manner as in Example 2, and as a result, the time required to complete the partial hydrogenation reaction was 50 minutes (including a 20- minute aging period).

The yield of 3,4-dihydrocoumarin formed by the partial hydrogenation reaction was 95% based on the coumarin.

### EXAMPLE 4

The ring formation and dehydrogenation reaction of methyl 3-(2-cyclohexanoyl)propionate was carried out in the same manner as in Example 2. Subsequently, instead of activating the catalyst in the reaction mixture, a catalyst consisting of active carbon, and 5% by weight of palladium supported thereon was added to the reaction mixture in an amount of 0.05% by weight based on the amount of the methyl 3-(2-cyclohexanoyl)propionate used. Using the resultant mixture, partial hydrogenation reaction was carried out in the same manner as in Example 2. The time required to complete the partial hydrogenation reaction was 70 minutes (including a 20-minute aging period).

The yield of 3,4-dihydrocoumarin formed by the partial hydrogenation reaction was 93% based on the coumarin.

### COMPARATIVE EXAMPLE 2

The ring formation and dehydrogenation reaction of methyl 3-(2-cyclohexanoyl)propionate was carried out in the same manner as in Example 2. The temperature of the reaction mixture was then adjusted to 80°C, and nitrogen gas containing no oxygen was bubbled through the reaction mixture for 30 minutes with stirring at 800 rpm. The resulting reaction mixture was brought into contact with hydrogen in order to conduct partial hydrogenation reaction, but the desired reaction did not proceed at all.

### COMPARATIVE EXAMPLE 3

The ring formation and dehydrogenation reaction of methyl 3-(2-cyclohexanoyl)propionate was carried out in the same manner as in Example 2. Without bringing the reaction mixture into contact with oxygen, the resulting reaction mixture was brought into contact with hydrogen in order to conduct partial hydrogenation reaction, but the desired reaction did not proceed at al.

## Claims

1. A process for producing a 3,4-dihydrocoumarin compound represented by formula (II): 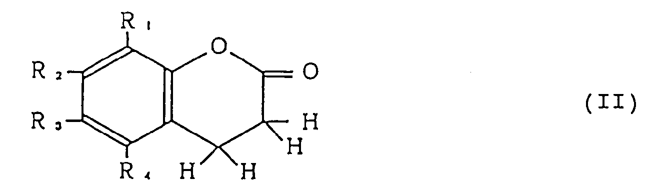 wherein R₁ to R₄ each represents a hydrogen atom, a methyl group or an ethyl group, provided that at least two of R₁ to R₄ each represents a hydrogen atom,
said process comprising the steps of:
(1) heating a 3-(2-cyclohexanoyl)propionic acid ester compound represented by formula (I): 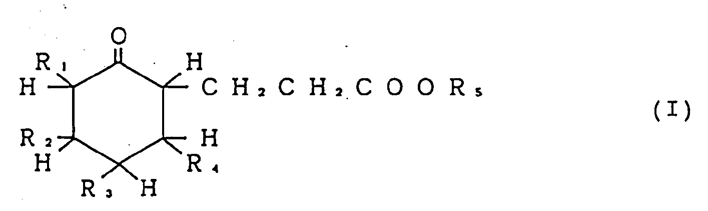 wherein R₁ to R₄ are as defined above, and R₅ represents an alkyl group having 1 to 4 carbon atoms,
in the presence of at least one solid metal catalyst selected from platinum, palladium, iridium, rhodium and nickel, thereby to allow said compound of formula (I) to undergo ring formation and dehydrogenation to yield said 3,4-dihydrocoumarin compound of formula (II) and, as a by-product, a coumarin compound represented by formula (III): 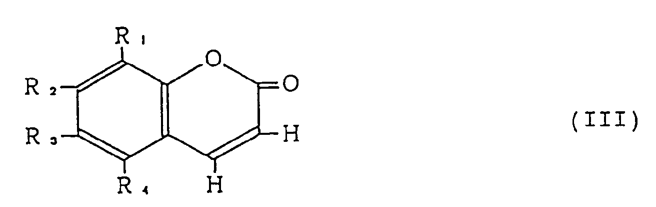 wherein R₁ to R₄ are as defined above; and
(2) partially hydrogenating said coumarin compound of formula (III),
said partial hydrogenation of said by-product coumarin compound of formula (III) being conducted, while said coumarin compound is kept unseparated from the reaction mixture that has undergone said ring formation and dehydrogenation,
by adding a catalyst which is as defined above to said reaction mixture, or bringing said reaction mixture into contact with oxygen to activate said catalyst used in said ring formation and dehydrogenation; and then bringing the resulting reaction mixture into contact with hydrogen thereby to partially hydrogenate said coumarin compound of formula (III) so as to convert said coumarin compound of formula (III) to 3,4-dihydrocoumarin compound of formula (II).

2. A process as claimed in Claim 1, wherein said 3-3-(2-cyclohexanoyl)propionic acid ester compound is methyl 3-(2-cyclohexanoyl)propionate.

3. A process as claimed in Claim 1 or 2, wherein said catalyst is palladium supported on active carbon.

4. A process as claimed in Claim 1,2 or 3, wherein the amount of the catalyst used for the ring formation and dehydrogenation is from 0.1 to 5% by weight based on the amount of the 3-(2-cyclohexanoyl)propionic acid ester compound.

5. A process as claimed in any of Claims 1 to 4, wherein the ring formation and dehydrogenation reaction is carried out at a temperature of from 100°C to 350°C.

6. A process as claimed in any preceding claim, wherein the amount of the catalyst added for the partial hydrogenation of the by-product coumarin compound is from 0.05 to 5% by weight based on the amount of the 3-(2-cyclohexanoyl) propionic acid ester compound.

7. A process as claimed in any preceding claim, wherein the oxygen which the reaction mixture is brought into contact with is pure oxygen gas or a gas containing oxygen.

8. A process as claimed in any preceding claim, wherein the contacting of the reaction mixture with oxygen is carried out at a temperature of from 0°C to 200°C.

9. A process as claimed in any preceding claim, wherein the partial hydrogenation reaction of the by-product coumarin compound is carried out at a temperature of from 80°C to 160°C.

## Patentansprüche

1. Verfahren zur Herstellung einer 3,4-Dihydrocumarinverbindung der Formel (II): 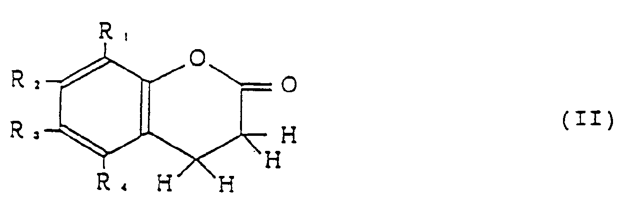 in der R₁ bis R₄ jeweils ein Wasserstoffatom, eine Methyl- oder Ethylgruppe darstellen, mit der Maßgabe, daß mindestens zwei der Reste R₁ bis R₄ jeweils ein Wasserstoffatom darstellen,
wobei das Verfahren die folgenden Schritte umfaßt:
(1) Erhitzen einer 3-(2-Cyclohexanoyl)propionsäureesterverbindung der Formel (I): 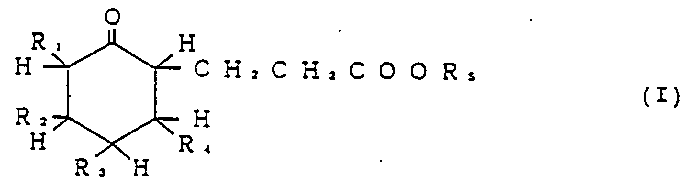 in der R₁ bis R₄ die vorstehend angegebene Bedeutung haben und R₅ einen Alkylrest mit 1 bis 4 Kohlenstoffatomen darstellt,
in Gegenwart mindestens eines festen Metallkatalysators, gewählt aus Platin, Palladium, Iridium, Rhodium und Nickel, wobei die Verbindung der Formel (I) unter Ringbildung und Dehydrierung, die 3,4-Dihydrocumarinverbindung der Formel (II) und, als Nebenprodukt, eine Cumarinverbindung der Formel (III) ergibt: 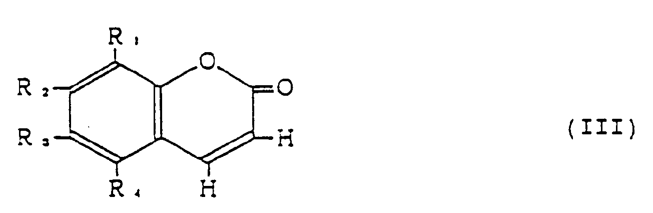 in der R₁ bis R₄ die vorstehend angegebene Bedeutung haben, und
(2) teilweise Hydrierung der Cumarinverbindung der Formel (III),
wobei die teilweise Hydrierung des Nebenprodukts, der Cumarinverbindung der Formel (III), so durchgeführt wird, daß die Cumarinverbindung nicht vom Reaktionsgemisch, das einer Ringbildung und Dehydrierung unterzogen wurde, abgetrennt wird und
daß man einen Katalysator, wie vorstehend definiert, zum Reaktionsgemisch gibt oder das Reaktionsgemisch mit Sauerstoff in Kontakt bringt, um den bei der Ringbildung und Dehydrierung verwendeten Katalysator zu aktivieren, und dann das entstehende Reaktionsgemisch mit Wasserstoff in Kontakt bringt, wobei die Cumarinverbindung der Formel (III) teilweise hydriert wird, sodaß die Cumarinverbindung der Formel (III) in die 3,4-Dihydrocumarinverbindung der Formel (II) umgewandelt wird.

2. Verfahren nach Anspruch 1, in dem die 3-(2-Cyclohexanoyl)propionsäureesterverbindung Methyl-3-(2-cyclohexanoyl)propionat ist.

3. Verfahren nach Anspruch 1 oder 2, in dem der Katalysator Palladium auf Aktivkohle ist.

4. Verfahren nach Anspruch 1, 2 oder 3, in dem die Menge des für die Ringbildung und Dehydrierung verwendeten Katalysators 0,1 bis 5 Gew.-%, bezogen auf die Menge der 3-(2-Cyclohexanoyl)propionsäureesterverbindung, beträgt.

5. Verfahren nach den Ansprüchen 1 bis 4, in dem die Ringbildung und Dehydrierungsreaktion bei einer Temperatur von 100°C bis 350°C durchgeführt wird.

6. Verfahren nach einem der vorstehenden Ansprüche, in dem die Menge des für die teilweise Hydrierung der als Nebenprodukt erhaltenen Cumarinverbindung zugegebenen Katalysators 0,05 bis 5 Gew.-%, bezogen auf die Menge der 3-(2-Cyclohexanoyl)propionsäuresterverbindung, beträgt.

7. Verfahren nach einem der vorstehenden Ansprüche, in dem der Sauerstoff, mit dem das Reaktionsgemisch in Kontakt gebracht wird, reines Sauerstoffgas oder ein sauerstoffhaltiges Gas ist.

8. Verfahren nach einem der vorstehenden Ansprüche, in dem das Inkontaktbringen des Reaktionsgemisches mit Sauerstoff bei einer Temperatur von 0°C bis 200°C durchgeführt wird.

9. Verfahren nach einem der vorstehenden Ansprüche, in dem die teilweise Hydrierungsreaktion der als Nebenprodukt erhaltenen Cumarinverbindung bei einer Temperatur von 80°C bis 160°C durchgeführt wird.

## Revendications

1. Procédé pour la préparation d'un composé 3,4-dihydrocoumarine représenté par la formule (II): 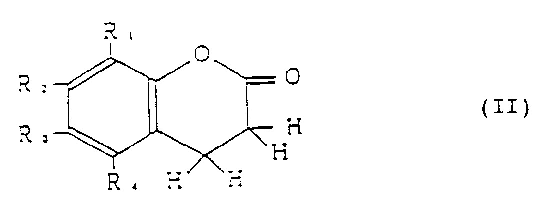 dans laquelle les groupes R₁ à R₄ représentent chacun un atome d'hydrogène, un groupe méthyle ou un groupe éthyle, à la condition qu'au moins deux des groupes R₁ à R₄ représentent chacun un atome d'hydrogène,
ledit procédé comprenant les étapes:
(1) de chauffage d'un composé ester de l'acide 3-(2-cyclohexanoyl)propionique représenté par la formule (I) 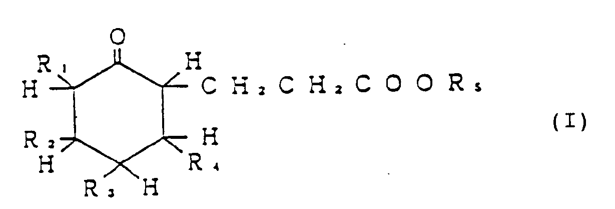 dans laquelle les groupes R₁ à R₄ sont tels que définis ci-dessus, et R₅ représente un groupe alkyle ayant de 1 à 4 atomes de carbone, en présence d'au moins un métal catalyseur solide choisi parmi le platine, le palladium, l'iridium, le rhodium et le nickel, grâce auquel ledit composé de formule (I) subit une formation de cycle et une déshydrogénation pour donner ledit composé 3,4-dihydrocoumarine de formule (II) et, en tant que sous-produit, un dérivé de coumarine représenté par la formule (III): 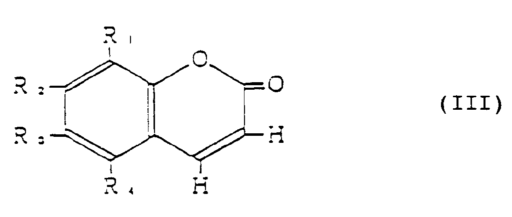 dans laquelle les groupes R₁ à R₄ sont tels que définis ci-dessus; et
(2) d'hydrogénation partielle dudit composé coumarine de formule (III), ladite hydrogénation partielle dudit composé sous-produit coumarine de formule (III) étant réalisée, alors que ledit composé coumarine n'est pas séparé du milieu réactionnel qui a conduit auxdites formation de cycle et déshydrogénation, en additionnant audit milieu réactionnel un catalyseur qui est tel que défini ci-dessus, ou en mettant ledit milieu réactionnel en contact avec de l'oxygène pour activer ledit catalyseur utilisé dans lesdites formation de cycle et déshydrogénation; puis en mettant en contact le milieu réactionnel résultant avec de l'hydrogène, et par ce moyen hydrogéner partiellement ledit composé coumarine de formule (III) de façon à convertir ledit composé coumarine de formule (III) en composé 3,4-dihydrocoumarine de formule (II).

2. Procédé selon la revendication 1, dans lequel ledit composé ester de l'acide 3-(2-cyclohexanoyl)propionique est le 3-(2-cyclohexanoyl)propionate de méthyle.

3. Procédé selon la revendication 1 ou 2, dans lequel ledit catalyseur est le palladium supporté par du charbon actif.

4. Procédé selon la revendication 1, 2 ou 3, dans lequel la quantité de catalyseur utilisé pour la formation de cycle et la déshydrogénation est de 0,1 à 5 % en poids par rapport à la quantité du composé ester de l'acide 3-(2-cyclohexanoyl)propionique.

5. Procédé selon l'une quelconque des revendications 1 à 4, dans lequel la réaction de formation de cycle et de déshydrogénation est réalisée à une température allant de 100°C à 350°C.

6. Procédé selon l'une quelconque des revendications précédentes, dans lequel la quantité de catalyseur additionné pour l'hydrogénation partielle du composé coumarine sous-produit est de 0,05 à 5 % en poids par rapport à la quantité du composé ester de l'acide 3-(2-cyclohexanoyl)propionique.

7. Procédé selon l'une quelconque des revendications précédentes, dans lequel l'oxygène avec lequel le milieu réactionnel est mis en contact est de l'oxygène gazeux pur ou un gaz contenant de l'oxygène.

8. Procédé selon l'une quelconque des revendications précédentes, dans lequel la mise en contact du milieu réactionnel avec l'oxygène est réalisée à une température allant de 0°C à 200°C.

9. Procédé selon l'une quelconque des revendications précédentes, dans lequel la réaction d'hydrogénation partielle du composé coumarine sous-produit est réalisée à une température allant de 80°C à 160°C.
